(19)

**Europäisches Patentamt**

**European Patent Office**

**Office européen des brevets**

(11) **EP 4 768 556 A1**

(12) **EUROPEAN PATENT APPLICATION**

(43) Date of publication:
01.07.2026 Bulletin 2026/27

(21) Application number: 25201539.1

(22) Date of filing: 11.09.2025

(51) International Patent Classification (IPC):
*C10G 11/18* $^{(2006.01)}$

(52) Cooperative Patent Classification (CPC):
**C10G 11/187; G16C 20/10;** C10G 2300/70;
G16C 20/70

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 27.12.2024 BR 102024027460

(71) Applicant: Petroleo Brasileiro S.A. - PETROBRAS
20031912 Rio de Janeiro (BR)

(72) Inventors:
• BASTIANI, RAQUEL
21941-915 Rio de Janeiro (BR)

• CHAMBERLAIN PRAVIA, OSCAR RENÉ
21941-915 Rio de Janeiro (BR)
• EFFTING, MARCEL
21941-915 Rio de Janeiro (BR)
• KNAPIK, ALLAN SILVESTRE
83900-000 São Mateus do Sul (BR)
• SILVA DE CASTRO, ANTONIO VICENTE
21941-915 Rio de Janeiro (BR)
• PINTO GONCALVES, RADHA LILIANE
21941-915 Rio de Janeiro (BR)

(74) Representative: Clarke, Modet y Cía., S.L.
C/ Suero de Quiñones 34-36
28002 Madrid (ES)

(54) **METHOD FOR PREDICTING THE PERFORMANCE OF A FLUID CATALYTIC CRACKING (FCC) UNIT**

(57) The present invention relates to a method for predicting the performance of a Fluid Catalytic Cracking (FCC) unit to optimize the operation of a FCC, which is essential for producing liquefied petroleum gas (LPG), propylene and naphtha, for example. The technical issue addressed herein is the difficulty in accurately predicting the yield, due to operational variables and catalyst deactivation, which affect the process efficiency. In this sense, the method includes: pre-training a model based on simulated data (BD_FCCSIM); refinement based on actual historical data (BD_Historica); fitting the model to consider process variables and catalyst properties; validation with error and correlation metrics; and implementation in a dynamic monitoring system that automatically adjusts the operational conditions. Such hybrid model, which combined physics and neural networks, provides accurate predictions and enables real-time adjustment, thereby enhancing the efficiency and adaptability of the FCC process in response to the market demand and operational conditions.

FIGURE 1

EP 4 768 556 A1

**Description**

**FIELD OF THE INVENTION**

The present invention falls within the technical field of chemical engineering and process technology, in particular, in the monitoring and optimization of fluid catalytic cracking (FCC) units in oil refineries.

The invention concerns a method for predicting the yield and performance of catalysts in FCC units, aiming to optimize the conversion of hydrocarbons into higher value products, such as LPG (Liquefied Petroleum Gas), propene, cracked naphtha, and other derivatives.

The invention is especially useful for refineries seeking to enhance the efficiency and precision of catalytic cracking operations, meeting variable market demands, as well as aiming to maximize catalyst performance over time.

**BACKGROUND OF THE INVENTION**

The goal of the invention is to address the limitations associated with predicting the performance of FCC units, wherein complex interactions between operational variables and catalyst properties are subject to frequent variations. These changes make it challenging to predict the yield and make real time adjustments of the operation.

Another challenge lies in the need to account for catalyst deactivation and the cumulative effects of contaminants, which directly affect yield efficiency and the quality of the final products.

In this sense, the present invention is intended to provide a predictive model that is capable of capturing the catalytic cracking process complexity by taking both the operational conditions and the specific characteristics of the catalyst used into account and that allows for dynamic fittings to be made in response to the market's demand and the current catalyst status, by leveraging physical modeling and machine learning techniques.

**STATE OF THE ART**

Document US11676061B2 describes a method that uses a real-time predictive model to control the sulfur level in gasoline produced by a fluid catalytic cracking (FCC) unit. It proposes adjusting the feed pre-treatment reactor temperature based on the sulfur levels measured in the final product (gasoline). The system uses machine learning and real-time data analysis to predict the sulfur level of gasoline based on the feed characteristics prior to treatment. In contrast, the present invention deals with a method of modeling the physicochemical properties of catalysts in order to optimize the yields of a catalytic cracking (FCC) unit, acting on the formulation of the catalyst to be fed to the FCC reactor. Furthermore, the automation system disclosed in document US11676061B2 is not implemented in a process simulator architecture capable of acting on multiple variables of the FCC process to meet product yield requirements. Furthermore, the automation system disclosed in said document is detailed to use a generic model that is not specified in the patent.

Document US10838412B2 describes systems and methods for improving the precision of olefin yield predictions in a cracking process (steam reformer - *steam cracker*) using a hybrid approach that combines machine learning techniques with phenomenological models. The less computationally demanding phenomenological model provides an initial prediction of olefin yield from a hydrocarbon stream, while machine learning corrects such prediction based on historical errors. To generate training data, a Design of Experiments (DOE) is used, in which both the phenomenological and detailed radical kinetic models are run under different conditions to identify discrepancies in the results. In contrast, the present invention deals with a method of modeling the physicochemical properties of catalysts in order to optimize the yields of a catalytic cracking (FCC) unit, acting on the formulation of the catalyst to be fed to the FCC reactor. The learning model used in document US10838412B2 is based on the use of outputs from two independent reaction model predictions: a phenomenological model and a free radical kinetic model. The difference in yield prediction between the models is determined and used to train a machine learning model. The machine learning model is then applied in real time, allowing the predicted yield to be corrected. That is, the machine learning model output serves to reposition the unit yields, without modifying the previous reaction models.

Document US11987758B2 discloses a method involving the development and implementation of a model for predicting the catalytic performance of residue FCC units that is applicable to any type of catalyst, including fresh catalysts and flushing catalysts (Purchased Ecat). This model has been integrated into process simulators such as PETRO-SIM™, FCC-SIM™ and HYSYS. The goal is to enhance the adherence of process simulators to the actual behavior of FCC units, in particular in regard to quality and quantity variations of flushing catalysts. By modifying existing simulators, the model ensures that these variations, which were previously not accurately predicted, can now be represented with greater fidelity, optimizing unit operation. In this regard, document US11987758B2 addresses the allocation of fresh and flushing catalysts without taking into account their formulation or physicochemical properties, aiming to optimize the flushing allocation across a set of FCC and RFCC units simultaneously, as proposed by the present invention. Fresh catalysts were mixed with flushing catalysts in any ratios, and the quality of the flushing catalyst used was changed for application in residue FCC units (RFCC). The differences in the catalysts were determined by catalytic tests in a pilot plant and the formulation or physical-chemical properties of the catalysts were not taken into account to estimate their model, nor was any form of machine learning modeling used, requiring expensive experimental resources that are not required in the present invention.

Document US11669063B2 refers to a method for modeling a chemical production process using simulations and machine learning models. The computer system described therein receives simulation event records, which contain inputs and outputs of a simulated chemical process. These records represent the mass flow and are limited by the physical characteristics of the equipment in an actual chemical production facility. The method consists of training a surrogate model, which can be a linear regression model, neural networks, among others, using simulation records. The surrogate model is trained to predict optimal outputs based on hypothetical inputs, and the inputs are limited by constraints such as the minimum and maximum values of the input variables observed in the simulation records.

Thus, document US11669063B2 describes a method to optimize the selection of an oil collection in a refinery by implementing constraints to the optimization outputs on surrogate models, which replace refining planning models, by constraining their output to production plans with current flow rates compatible with the refinery unit capacities. For example, if the plant's production capacity is 1000 gallons/hour of jet fuel, and the surrogate model finds a solution of 1200 gallons/hour, this means that the model needs to be retrained based on new results from the traditional simulation (refinery planning model), since the simulated model has this clear constraint and the surrogate model does not. However, document US11669063B2 does not apply to the modeling of a process unit, nor to the modeling of catalyst properties for the performance response of a FCC unit, thus teaching away from the present invention.

The paper "Artificial Intelligence for Hybrid Modeling in Fluid Catalytic Cracking (FCC)" deals with a model for predicting FCC yields from a 3D Computational Particle Fluid Dynamics (CPFD) study and FCC process data to train and validate a machine learning (ML) model. CPFD is used to simulate the behavior of fluids and particles in FCC reactors, allowing the analysis of complex operating conditions on an industrial scale. The document does not describe the optimization method, but rather the direct prediction of plant results. Said document studies the interaction of equipment geometries and the interaction between the fluid phase and catalyst particles, without specifying the physicochemical properties of the catalyst and their formulation. The output of hybrid ML-CFD modeling serves to accelerate CFD modeling results. In contrast, the present invention relies on the prediction of a large database simulated by a phenomenological FCC process simulator to pre-train a machine learning model and then model is retrained with actual data contemplating the physicochemical properties of the catalysts. The result is a unique modeling that is capable of predicting and optimizing different FCC catalyst formulations, resulting in a cost-effective optimization of the catalyst formulation, which step is not present in this paper.

Thus, it is evident that none of the cited prior-art documents anticipate a prediction method comprising a hybrid model trained with both simulation data and actual data to optimize the formulation of catalysts, and which is capable of predicting an industrial plant performance in a more accurate and cost-effective manner, as proposed by the present invention.

## SUMMARY OF THE INVENTION

The present invention provides a a predictive method to optimize the performance of a catalytic cracking unit (FCC), which is essential for the production of LPG (Liquefied Petroleum Gas), propylene and naphtha, for example. The technical problem addressed herein is the difficulty in accurately predicting FCC unit yields due to the physical-chemical properties of the fresh catalytic system along with the operational variables and catalyst deactivation, which affect the process output. The method of the present invention includes: pre-training a model using simulated data (BD _FCCSIM - referring to the first database); refining using actual historical data (BD_Histórica - referring to the second database); fitting the model to account for process variables and physicochemical properties of the catalysts; validating using error and correlation metrics; and implementing in a monitoring system that incorporates the changes to the hybrid model in the process simulator, thereby enabling real time optimization of the unit's catalytic system along with operational conditions. This method, which contemplates the hybrid model adapted to the process simulator, combines phenomenological modeling and neural networks to provide accurate predictions and enable real-time fittings, thereby enhancing the efficiency and adaptability of the FCC process in response to the market demand and operational conditions.

## BRIEF DESCRIPTION OF THE DRAWINGS

The present invention will be described below with reference to its typical embodiments and also with reference to the appended drawings, in which:

Figure 1 is a flowchart of the present invention illustrating the steps of the method.

Figure 2 is a graphical representation of the fitting of the hybrid model for fuel gas yield expressed in mass percent, in accordance with an exemplary embodiment of the present invention.

Figure 3 is a graphical representation of the fitting of the hybrid model for hydrogen yield expressed in mass percent, in accordance with an exemplary embodiment of the present invention.

Figure 4 is a graphical representation of the fitting of the hybrid model for LPG yield expressed in mass percent, in accordance with an exemplary embodiment of the present invention.

Figure 5 is a graphical representation of the fitting of the hybrid model for propene yield expressed in mass percent, in accordance with an exemplary embodiment of the present invention.

Figure 6 is a graphical representation of the fitting of the hybrid model for cracked naphtha yield expressed in mass percent, in accordance with an exemplary embodiment of the present invention.

Figure 7 is a graphical representation of the fitting of the hybrid model for LCO (Light Cycle Oil) yield expressed in mass percent, in accordance with an exemplary embodiment of the present invention.

Figure 8 is a graphical representation of the fitting of the hybrid model for decanted oil yield expressed in mass percent, in accordance with an exemplary embodiment of the present invention.

Figure 9 is a graphical representation of the fitting of the hybrid model for coke yield expressed in mass percent, in accordance with an exemplary embodiment of the present invention.

## DETAILED DESCRIPTION OF THE INVENTION

The present invention relates to a method of predictive modeling that combines phenomenological modeling and Multilayer Perceptron (MLP) neural networks to simulate and predict the performance of a FCC unit. The method comprises several sequential steps that involve pre-training the model with a simulated database, refinement with a historical database of the FCC unit, fitting and setting the model to predict the unit yields based on the characteristics of the catalyst, validating the model based on error and correlation metrics, and finally, implementing the hybrid model in the process simulator to provide a monitoring and optimization system for the FCC unit by simultaneously adjusting the catalytic system and the unit's conditions.

Pre-Training of the Predictive Model

The first step of the method consists of pre-training a predictive model using a simulated database (BD_FCCSIM). This database is generated using a FCC-specific simulator, which represents different operational scenarios that integrates different process parameters and catalyst characteristics.

The BD_FCCSIM database includes input variables that represent the operational conditions of the FCC unit, such as ZSM-5 zeolite content, reaction temperature (RXT), dense phase temperature (DPT), dilute phase temperature (DilPT), combined feed temperature (CFT), feed flow rate ($F_{Feed}$), vacuum heavy gas oil flow rate ($F_{GOP}$), coke heavy gas oil flow rate (VGopk), naphtha riser flow rate (NRF), 20/4 density of vacuum heavy gas oil ($d20_{Feed}$), 20/4 density of coke gas oil ($d20_{Gopk}$), basic feed nitrogen ($BN_{Feed}$), and specific properties of fresh catalysts, such as specific area (VCatSA), pore volume (VCatPV), bulk density (VCatBD), and metal content such as sodium (VCatNa), iron (VCatFe), and rare earths (VCatRE). Furthermore, such database considers equilibrium catalyst (Ecat) variables, including activity, coke content, accessibility, and contaminant content, such as vanadium and nickel.

To generate the BD_FCCSIM, a factorial simulation design is applied, which enables the creation of multiple scenarios with variations in the operational conditions and catalyst parameters. Such planning must be carried out in more than one distinct period, each with a different calibration. In both periods, a common base catalyst is used, with one period preferably including the ZSM-5 zeolite additive, while the other preferably does not. This approach allows the predictive model to learn the influence of different compositions and process conditions on the FCC unit performance.

During pre-training, the MLP model is configured with training and testing data sets from the simulated base BD_FCCSIM. Overfitting prevention techniques, such as early stopping, are applied to ensure that the model does not overfit the pre-training data, thereby maintaining a better generalization for the next step.

Refinement using Historical Database (BD Histórica)

After pre-training, the predictive model is refined using a historical database (BD_Histórica) comprising actual data from the FCC unit. Such database covers an extended period of unit operation, including process variables, fresh and equilibrium catalyst (Ecat) parameters, and product yields. The BD_Histórica contains yield data for products such as fuel gas, LPG, cracked naphtha, LCO, decanted oil, and coke, as well as gas chromatography variables (hydrogen and C1 to C4), CO and $CO_2$ content in the flue gases, reaction temperature, feed density and GOP flow rate. This data allows the model to incorporate actual conditions of the unit, reflecting the influences of contaminants on the catalyst and operational variables.

To ensure data consistency and quality, BD_Histórica is filtered to delete records where the mass balance falls outside the 98 to 102% range and to exclude data entries with missing values. The data is then divided into three sets: training, testing, and validation, using data from the same two months of each year to compose the validation set. Such refinement fits the MLP model to consider characteristics of the catalysts while in use, fitting it to improve the prediction accuracy and the representativeness of optimizations.

Fitting and Setting the Model

The next step involves fitting and setting the MLP neural network to predict FCC unit yields and characteristics of catalysts in use. The model is structured with one input layer, two hidden layers and one output layer. The input layer receives the operational variables and catalyst characteristics, while the hidden layers are set with between 50 to 200 neurons, depending on the parameters that minimize the root mean square error (RMSE) and maximize the correlation coefficient ($R^2$) for the testing and validation data. The activation functions used may include "Rectified Linear Unit" (ReLU) and hyperbolic tangent types, with normalized weight initialization to optimize the learning process.

To ensure that the model maintains its accuracy when predicting yields, the mass balance is standardized to 100% prior to and after the modeling process, and the input and output variables are normalized to the range of 0 to 1. Normalizing and adjusting the batch size to 10 samples per iteration optimizes the training process, ensuring the effective model learning and reducing the need for fitting when applied to subsequent data.

Model Validation

The validation stage is essential to ensure the accuracy and applicability of the predictive model. The model is validated based on two main metrics: the Root Mean Square Error (RMSE) and the coefficient of determination ($R^2$), applied to the test and validation data sets. RMSE measures the accuracy of predictions, while $R^2$ shows the strength of the correlation between the predicted values and actual values, providing insight into how effective the model is in explaining variations in the data.

Furthermore, validation is performed with multiple training and testing iterations to calculate the mean and standard deviation of the RMSE and $R^2$ values, ensuring consistent results. Comparison between the RMSE and $R^2$ values obtained in the pre-training (simulated values) and validation (actual values) datasets ensures that the model can be applied to both simulated and actual conditions by selecting the setting with the lowest RMSE and the highest $R^2$.

Finally, the predictive model is implemented in an FCC unit monitoring system. This system integrates the model into the unit in real-time, enabling the continuous collection of operational data and using model predictions to adjust the catalytic system formulation in accordance with operational conditions.

EXEMPLARY EMBODIMENT

In an exemplary embodiment, only the pre-trained MLP model was selected as an example for predicting the yield and *Catalyst Factors* (CFs) for CDB/FCC-SIM™, based on the properties of fresh catalysts commercially used in the U-220 unit of the REPLAN refinery.

The following models used two databases. The first database was generated from a factorial design that considered the catalyst and process parameters and the yields achieved through the FCC-SIM™ process simulator. This planning was carried out with data from two different dates; that is, two different calibrations in the process simulator. The chosen periods have the same base catalyst, but one period used ZSM-5 additive, while the other did not. The response variables were modeled in the FCC-SIM™ simulator according to the full factorial design listed in Table I. In this design, 11,664 cases were simulated for each calibration: 1) calibration from June 15, 2022, using a catalyst formulation containing 1.5% ZSM-5 and replacement of 4.5 t/d; 2) calibration from April 26, 2017 using a formulation with no ZSM-5 and a replacement of 7.5 t/d. Therefore, the full pre-training database of the model contains 23,328 cases.

Table I - Factorial design of simulated cases in FCC-SIM™.

| Variable | Minimum value | Maximum value | Step | Levels |
|---|---|---|---|---|
| CFT | 220 | 280 | 30 | 3 |
| RXT | 525 | 540 | 7.5 | 3 |
| DPT | 690 | 720 | 30 | 2 |
| NRF | 0 | 2000 | 1000 | 3 |
| Vgopk | 0 | 2000 | 1000 | 3 |
| $V_{feed}$ | 7300 | 7800 | 500 | 2 |
| $d20_{Feed}$ | 0.92 | 0.945 | 0.0125 | 3 |
| $NB_{Feed}$ | 800 | 1500 | 700 | 2 |
| ECatV | 750 | 1500 | 375 | 3 |
| ECatNa | 0.4 | 0.601 | 0.2 | 2 |

The second database contains the actual data from REPLAN's U-220 unit spanning the period from January 2011 to June 2022, and contains information on the unit's parameters, the fresh catalyst and the equilibrium catalyst (Ecat) and their respective yields. The yields of fuel gas, LPG, cracked naphtha, LCO, decanted oil, and coke were considered in the analyses. Furthermore, gas chromatography was considered, covering hydrogen and the range of from C1 to C4.

Unit parameters: CO content in flue gases (FGCO), $CO_2$ content in flue gases (FGCO2), 20/4 density of the feed (d20Feed), 20/4 density of coke gas oil (d20Gopk), basic nitrogen of the feed (NBFeed), riser 1 reaction temperature (RXT), riser 2 reaction temperature (RXT2), dense phase temperature (DPT), dilute phase temperature (DilPT), combined feed temperature (CFT), blower air flow rate (AirBlow), feed flow rate ($F_{Feed}$), GOP flow rate ($F_{GOP}$), GOPK flow rate (VGopk), riser naphtha flow rate (RNF), and catalyst replacement (RepCat);

Fresh catalyst parameters: catalyst specific area (VCatSA), deactivated catalyst specific area (788 °C/100% steam for 5 h) (VCatDSA), catalyst pore volume (VCatPV), catalyst bulk density (VCatBD catalyst unit cell size (VCatUCS), catalyst accessibility (VCatAAI), catalyst alumina content (VCatAl), sodium content (VCatNa), iron content (VCatFe), rare earth content (VCatRE), phosphorus content (VCatP), deactivated catalyst activity (788 °C/100% steam for 5 h) (VCatMAT), and ZSM-5 zeolite content (ZSM); and

Equilibrium catalyst (Ecat) parameters: Ecat activity (ECatMAT), Ecat accessibility (ECatAAI), Ecat specific area (ECatSA), Ecat micropore volume (ECatMiPV), Ecat mesopore area (ECatMSA), Ecat coke content (ECatK), Ecat alumina content (ECatAl), Ecat rare earth content (ECatRE), Ecat sodium content (ECatNa), Ecat iron content (ECatFe), Ecat vanadium content (ECatV), Ecat nickel content (ECatNi), Ecat phosphorus content (ECatP), and Ecat platinum content (ECatPt).

The databases used in this study were designated as follows:

- BD_FCCSIM: Referring to the first database, which consists of the yields achieved by simulations using the factorial design recited in Table I (cases: 23,328).

- BD_Histórica: Referring to the second database, which covers the period from January 2011 to June 2022 and contains the unit data, including fresh catalyst and Ecat parameters as well as process parameters with their respective yields over that period. The strategy adopted was to filter the data for which the unit material balance fell outside the range of 98 to 102 and for cases with missing data. Accordingly, the original database with 4,974 cases was reduced to 2,918 cases; however, quality of the data information improved.

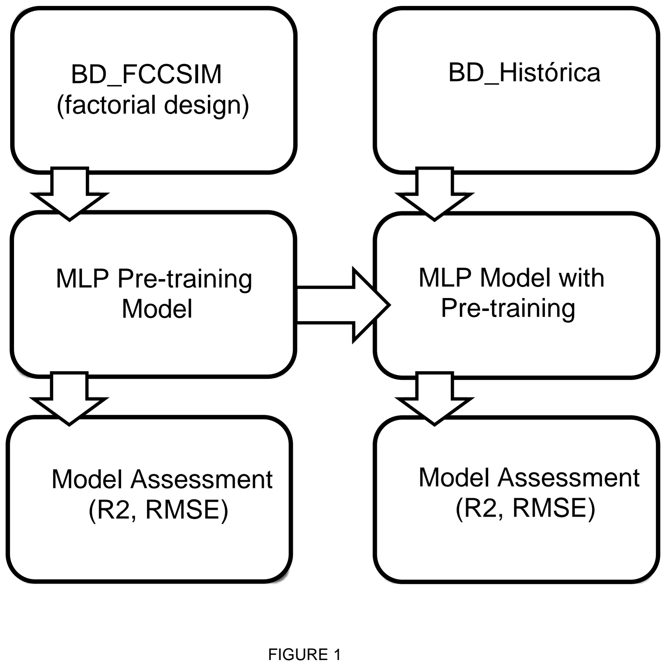

These two databases were used to perform the analyses and develop the necessary models. The goal is to combine phenomenological modeling with machine learning. This approach seeks to take advantage of both methods, using phenomenological modeling to capture prior knowledge and existing physicochemical relationships, while machine learning is used to deal with the complexity and uncertainty of the systems. Therefore, this approach allows for the consideration of the physicochemical laws underlying the studied systems. Accordingly, estimation of neural network parameters follows the flowchart in Figure 1.

The Pre-Training MLP (Multilayer Perceptron) model uses the BD_FCCSIM database of factorial design for simulation with FCC-SIM™ (Table I). This approach incorporates partial physical and kinetic elements to define the network parameters, which will later be refined with actual data from BD_Histórica. The data was divided into training (70%) and testing (30%) sets.

The fresh catalyst properties were represented solely by the composition of two catalysts, only differing in the ZSM-5 zeolite content. The equilibrium catalyst contaminant metal parameters (Ecat) and process variables were assessed as independent variables, and the yields and C1-C4 chromatography as dependent variables. The mass balance of the yields was standardized to 100 before and after modeling. Input and output variables were normalized to the range of 0 to 1. To prevent overfitting, the early stopping technique was applied. The batch size was set at 10, without considering computational time constraints.

The proposed MLP model has 26 input variables (process, fresh catalyst and Ecat contamination) and 19 output variables (yields and chromatography). The input layer consists of an initialization layer that receives 26 input variables and the bias and used normal initialization models and Rectified Linear Unit (ReLU) activation function (kernel_initializer package='normal', activation='relu'), and contains 28 neurons. Two more hidden layers were used with normal initialization and hyperbolic tangent activation function (kernel_initializer='normal', activation='tanh') and with a varying number of neurons depending on the architecture assessed. The output layer is a combination of the 19 modeled variables and is represented by normal initialization models (kernel_initializer='normal'). Description of the strategy employed in each MPL carried out is presented in Table II. The evaluation and validation criterion of the models was the Root Mean Square Error (RMSE) when validating the model, analyzing different numbers of neurons (50, 100, 200) in the two different hidden layers. Five different training sessions were carried out to obtain the average RMSE and the correlation coefficient ($R^2$) for each hyperparameter choice. Table II also shows the average RMSE of the main yields and the coefficient of correlation ($R^2$) of the trained models. The models obtained exhibited higher $R^2$ values and lower RMSEs for the individual yields when using 50 neurons in the hidden layers, except for cracked naphtha yield, where the 100 neuron-model achieved the lowest RMSE. Since the data generating this pre-trained MLP model was based on simulated data, errors are small.

The parameters in Table II are defined as follows: 26 input variables encompassing process and catalyst variables; 19 output variables comprises main yields and C1 to C4 gases.

Considering an example with 50 neurons in the hidden layers, the parameters of each layer are defined as follows:

- Initialization layer (input layer):

$$(26 \text{ input variables} + 1 \text{ bias}) \text{ x (input layer (28))}$$

$$= 756 \text{ (Eq. 1)}$$

- Dense layer 1 is given by:

$$(\text{input layer (28)} + 1 \text{ bias}) \text{ x Dense layer1 (50)} = 1450$$

$$(\text{Eq. 2})$$

- Dense layer 2 is given by:

$$(\text{Dense layer1 (50)} + 1 \text{ bias}) \text{ x Dense layer2 (50)} = 2550$$

$$(\text{Eq. 3})$$

- Output layer:

$$(\text{Dense layer2 (50)} + 1 \text{ bias}) \text{ x 19 output variables} = 969 \text{ (Eq. 4)}$$

Table II - RMSE and $R^2$ of the test versus the number of neurons for the pre-training MLP model (BD _FCCSIM)

| | Neurons | Parameters | Neurons | Parameters | Neurons | Parameters |
|---|---|---|---|---|---|---|
| Model | 50n | | 100n | | 200n | |
| Input | 28 | 756 | 28 | 756 | 28 | 756 |
| Dense 1 | 50 | 1450 | 100 | 2900 | 200 | 5800 |
| Dense 2 | 50 | 2550 | 100 | 10100 | 200 | 40200 |

(continued)

| | Neurons | Parameters | Neurons | Parameters | Neurons | Parameters |
|---|---|---|---|---|---|---|
| Model | 50n | | 100n | | 200n | |
| Output | 19 | 969 | 19 | 1919 | 19 | 3819 |
| Total | | 5725 | | 15675 | | 50575 |
| | Average | STD | Average | STD | Average | STD |
| $R^2$ train | 0.9989 | 0.0001 | 0.9986 | 0.0002 | 0.9987 | 0.0005 |
| $R^2$ test | 0.9989 | 0.0001 | 0.9986 | 0.0002 | 0.9987 | 0.0005 |
| RMSE GC (%m) | 0.0210 | 0.0029 | 0.0253 | 0.0039 | 0.0198 | 0.0039 |
| RMSE GLP (%m) | 0.1177 | 0.0106 | 0.1289 | 0.0131 | 0.1352 | 0.0246 |
| RMSE NC (%m) | 0.1706 | 0.0422 | 0.1893 | 0.0343 | 0.2117 | 0.0981 |
| RMSE LCO (%m) | 0.1125 | 0.0117 | 0.1308 | 0.0171 | 0.1252 | 0.0181 |
| RMSE OD(%m) | 0.1264 | 0.0133 | 0.1368 | 0.0158 | 0.1449 | 0.0388 |
| RMSE Coke (%m) | 0.0277 | 0.0031 | 0.0322 | 0.0037 | 0.0281 | 0.0034 |

Although the RMSE variation between the number of neurons is reduced, the network structure with 50 neurons in the inner layers was chosen for the MLP models.

When building the MLP model with pre-training, the historical database, BD_Histórica, was used, together with the structure of the pre-training MLP model achieved in the prior section. In this step, the network parameters are refined with actual data from REPLAN's U-220 unit using its database (BD_Histórica). The database was divided into three sets, a training set (58.3%), a testing set (25.0%) and a validation set (16.7%, corresponding to the months of June and July of each year). Training and testing data are randomized by the model's own strategy.

In this step, the fresh catalyst properties were represented, as well as the replacement of fresh catalyst and the Ecat contamination by contaminating metals, and the process variables were assessed as independent variables. The yields and chromatography of C1-C4 gases as dependent variables. The mass balance of the yields was standardized to 100 before and after modeling. Input and output variables were normalized to the range of 0 to 1. To prevent overfitting, the early stopping technique was applied. Batch size was reduced to 2 since the database is smaller compared to the one used for pre-training. The MLP model re-estimated its parameters from the best model obtained in the previous step (Pre-Training MLP Model based on FCCSIM™). That is, the MLP model has the same parameters and initiation and activation functions.

The evaluation and validation criterion of the models was RMSE (*Root Mean Square Error*) in model validation, for 50 neurons in both inner layers. Ten iterations were performed to obtain the average RMSE and the coefficient of correlation ($R^2$). Table III further shows the RMSE of the interactions and the coefficient of correlation ($R^2$) of the model. The models converged between 11 and 39 epochs, even though the algorithm was provided with 200 epochs.

Table III - RMSE and $R^2$ of the MLP model with pre-training and data from the U-220 unit (BD-Histórica)

| Model | Training | Test | Validation |
|---|---|---|---|
| | RMSE | RMSE | RMSE |
| 0 | 0.668 | 0.670 | 0.784 |
| 1 | 0.643 | 0.651 | 0.755 |
| 2 | 0.642 | 0.646 | 0.729 |
| 3 | 0.634 | 0.641 | 0.794 |
| 4 | 0.628 | 0.637 | 0.771 |
| 5 | 0.618 | 0.629 | 0.779 |
| 6 | 0.614 | 0.628 | 0.778 |
| 7 | 0.595 | 0.615 | 0.791 |
| 8 | 0.604 | 0.621 | 0.766 |

(continued)

| Model | Training | Test | Validation |
|---|---|---|---|
| | RMSE | RMSE | RMSE |
| 9 | 0.602 | 0.621 | 0.808 |

Table II - RMSE of the yields of MLP models with pre-training and data from the U-220 unit (BD-Historical)

| Model | RMSE GC | RMSE H$_2$ | RMSE GLP | RMSE Propene | RMSE NC | RMSE LCO | RMSE OD | RMSE Coke |
|---|---|---|---|---|---|---|---|---|
| 0 | 0.417 | 0.0123 | 1.264 | 0.614 | 1.811 | 1.339 | 1.950 | 0.247 |
| 1 | 0.409 | 0.0124 | 1.203 | 0.575 | 1.683 | 1.380 | 1.875 | 0.270 |
| 2 | 0.437 | 0.0125 | 1.350 | 0.560 | 1.673 | 1.338 | 1.558 | 0.253 |
| 3 | 0.429 | 0.0122 | 1.453 | 0.632 | 1.668 | 1.402 | 1.931 | 0.255 |
| 4 | 0.429 | 0.0118 | 1.250 | 0.550 | 1.845 | 1.343 | 1.843 | 0.241 |
| 5 | 0.441 | 0.0127 | 1.199 | 0.536 | 1.866 | 1.312 | 1.950 | 0.255 |
| 6 | 0.413 | 0.0129 | 1.298 | 0.589 | 1.876 | 1.351 | 1.801 | 0.261 |
| 7 | 0.440 | 0.0125 | 1.404 | 0.617 | 1.821 | 1.361 | 1.861 | 0.257 |
| 8 | 0.442 | 0.0125 | 1.172 | 0.550 | 1.932 | 1.333 | 1.741 | 0.255 |
| 9 | 0.419 | 0.0125 | 1.314 | 0.566 | 1.979 | 1.344 | 1.927 | 0.252 |

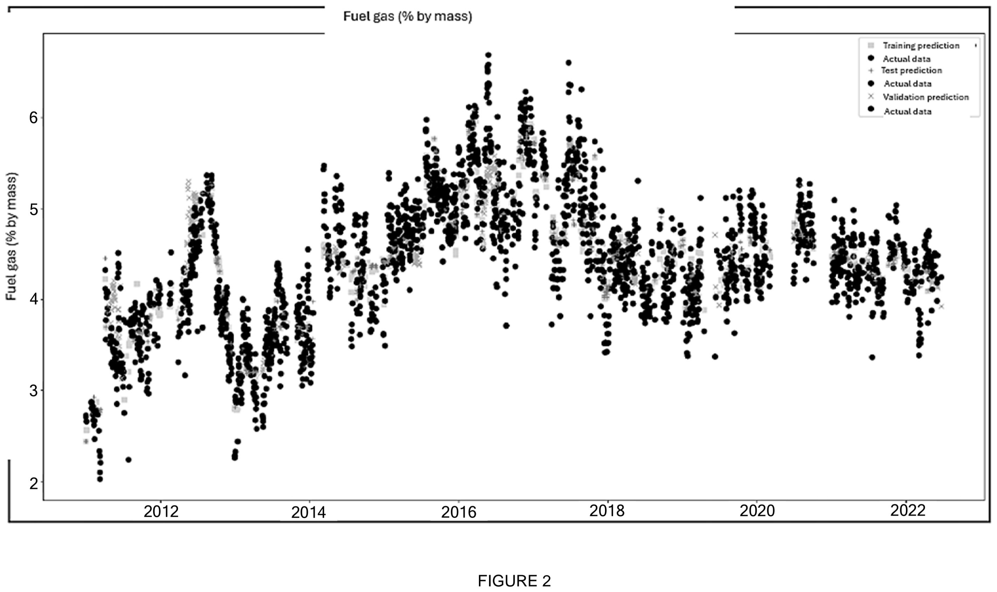

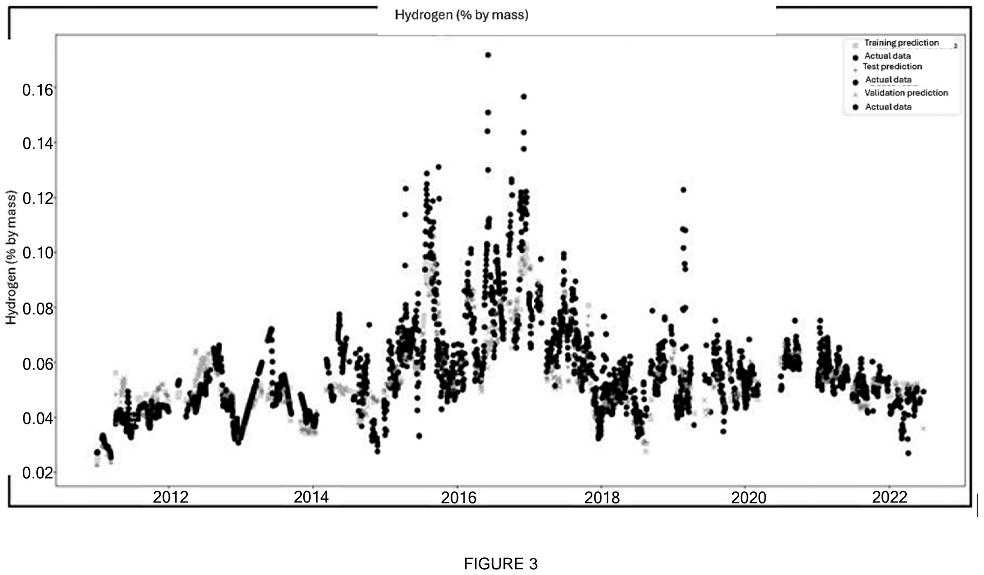

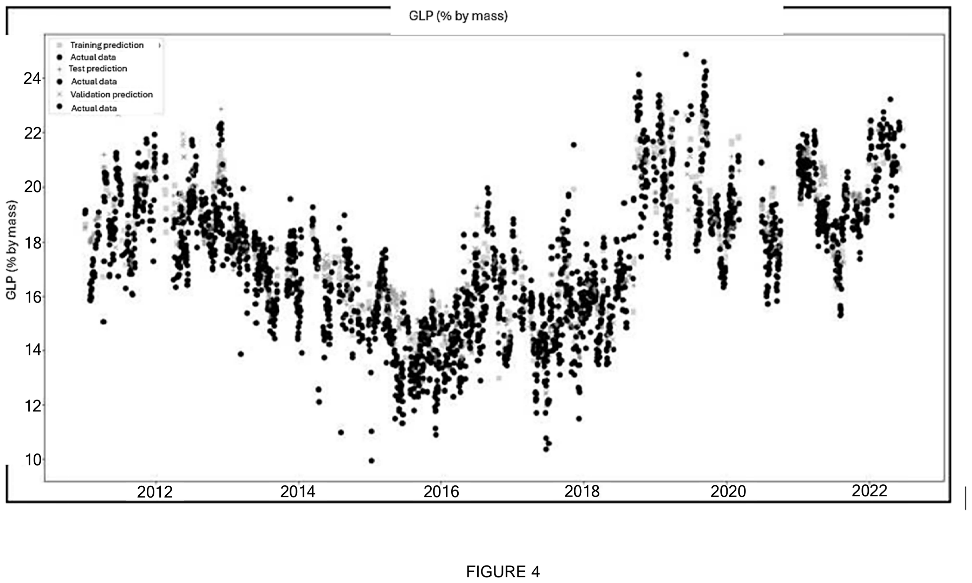

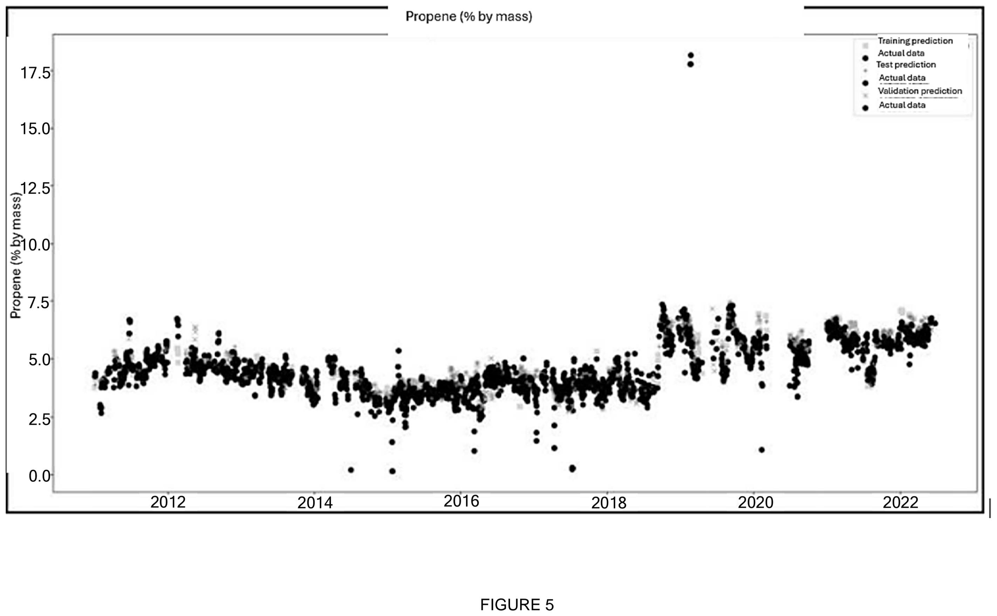

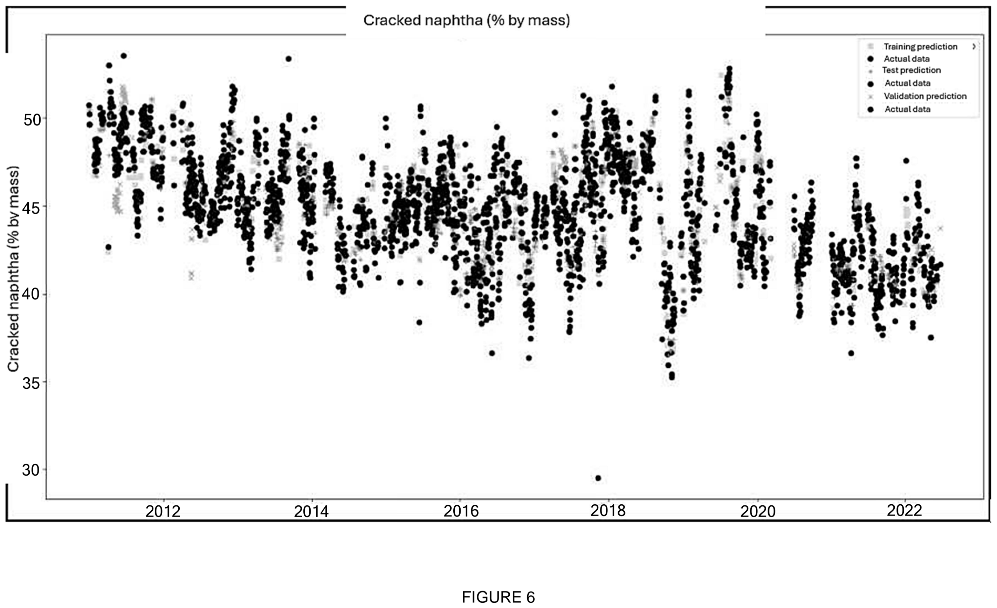

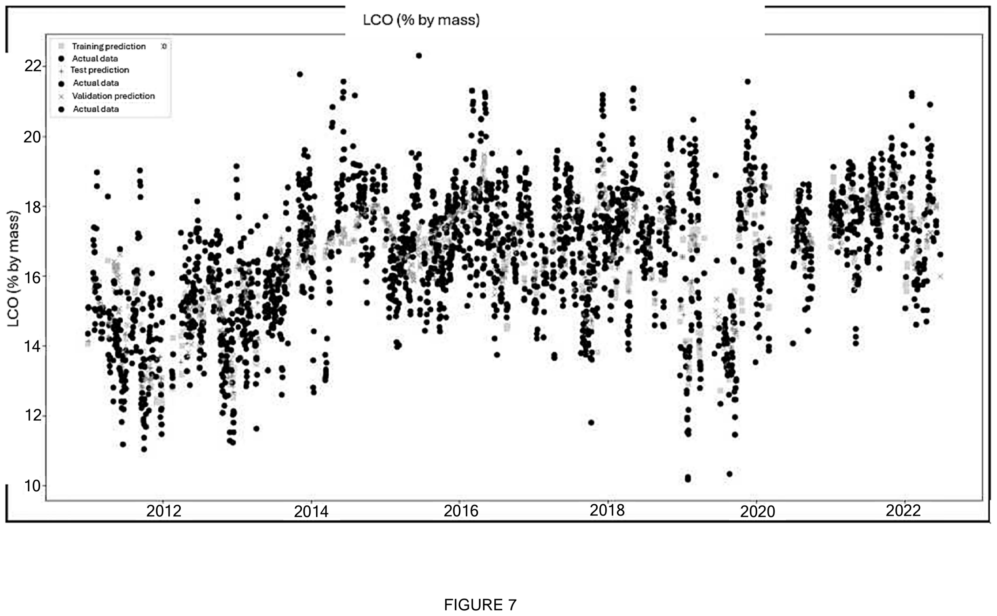

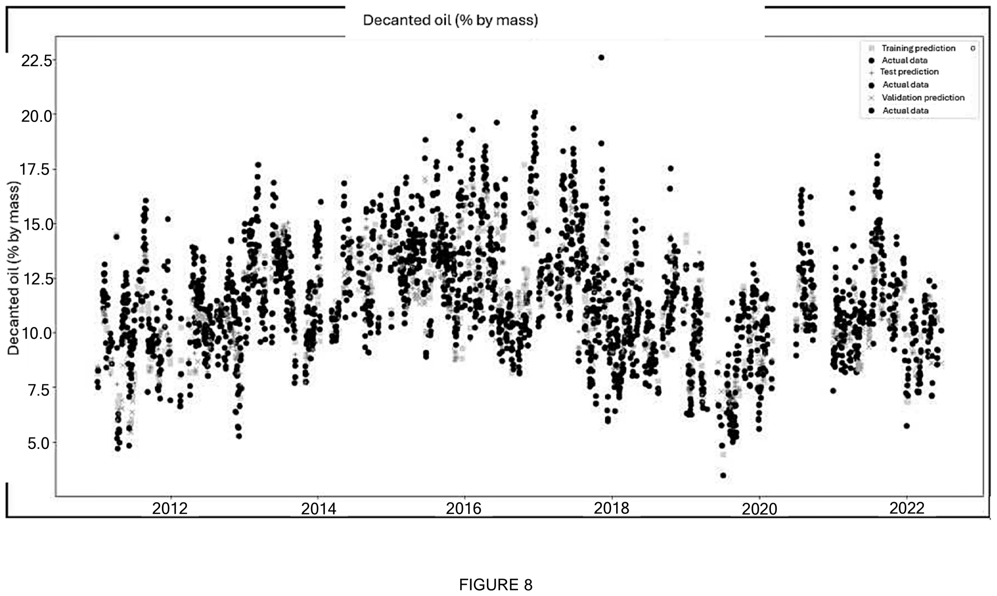

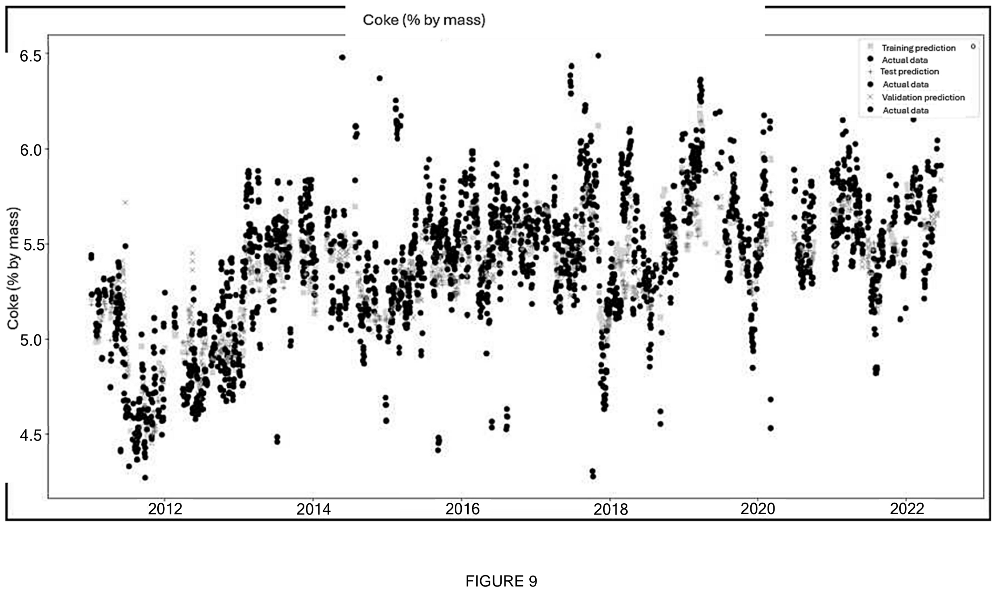

Figures 2 to 9 illustrate the model fitting to the actual data, wherein: the black dots represent actual data from the U-220 unit; the gray square dots represent the training prediction; the grayer dots represent the test prediction; and the gray cross dots represent the validation prediction. More specifically, Figure 2 shows the model results for fuel gas yield, Figure 3 shows the model results for hydrogen yield, Figure 4 shows the model results for LPG yield, Figure 5 shows the model results for propene yield, Figure 6 shows the model results for cracked naphtha yield, Figure 7 shows the model results for LCO yield, Figure 8 shows the model results for decanted oil yield, and Figure 9 shows the model results for coke yield. It should be noted that although the validation model assumes low $R^2$ values, the graph shows that the data is highly dispersed, and the model predicted data falls within this range for all yields. High dispersion of the experimental data did not allow a better value of $R^2$ for validating the models.

Comparison of the performance prediction between the Hybrid MLP model and industrial catalyst findings from U-220

A comparison between the industrial findings of existing catalysts by switching catalytic systems in U-220 with the prediction of the Hybrid MLP model developed herein was carried out. The commercial evaluations carried out followed the PETROBRAS standard for industrial catalyst assessment (BASTIANI, R. et al. Padrão de Avaliação Industrial de Catalisadores. Rio de Janeiro: PETROBRAS. CENPES. PDAB. TFCC, 2011. 76f Technical Communication (CT TFCC No. 007/2011). Comparison of the catalyst performance is presented in terms of delta yields between the assessed catalysts and, according to the standard, such an assessment can be carried out by: 1. Group analysis, when there is a group with both catalysts under similar operational conditions and according to the PETROBRAS standard for assessment; 2. Pure MLP Neural Networks with data from the period during which the catalysts are being assessed; 3. Assessment of Ecats of the catalytic systems in laboratory units (ACE unit: 535 °C, 100% GOP) and/or pilot plant (DCR unit: 540 °C, 100% GOP). In industrial assessments of FCC catalytic systems, the catalyst performance ranking is sought. To this end, assessment techniques are applied; and, whenever possible, the greatest possible agreement between them is sought.

Prediction of the Hybrid MLP model was performed in three ways: 1. Prediction of catalytic performance for the same catalytic systems assessed in the industrial assessment, considering the average performance for the operation with 4974 data; that is, for the entire operating period on which the model estimate was based (January 2011 to June 2022, REPLAN unit U-220); 2. Prediction of catalytic performance for the same catalytic systems assessed in the industrial assessment, only considering the period in which the catalysts are being assessed; 3. Catalyst Factors (CFs) calculated from the prediction of the Hybrid MLP model for the catalytic systems used in U-220, ans such CF were fed into the CDB/FCC-SIM™ model in order to modify the CDB factors, thereby changing the FCC-SIM™ riser/reactor expressions that predict product yields and properties under different catalytic systems. This approach, therefore, can predict the performance resulting from a change in the catalytic system from any calibration of the U-220 unit within the FCC-SIM™ software. Table V shows the characterization of fresh catalysts used to predict and estimate catalyst CFs by the hybrid MLP model for catalytic systems used in REPLAN U-220 unit from January 2011 to June 2022.

Table V - Characterization of fresh catalysts used for predicting and estimating CFs of the hybrid MLP model.

| Catalyst | SIRIUS | SIRIUS | SIRIUS |
|---|---|---|---|
| | 2727 | 2147 | ZOOM 2176 |
| Specific area catalyst ($m^2/g$) | 316 | 313 | 312 |
| Specific area of the deactivated catalyst ($m^2/g$) | 180 | 176 | 175 |
| Pore volume (ml/g) | 0.412 | 0.409 | 0.412 |
| Bulk density (g/ml) | 0.74 | 0.75 | 0.75 |
| Unit cell size (nm) | 2.460 | 2.460 | 2.460 |
| Accessibility (%) | 8.2 | 11.7 | 11.0 |
| Alumina content (% w/w) | 49.35 | 53.68 | 52.36 |
| Sodium content (% w/w) | 0.35 | 0.39 | 0.35 |
| Iron content (% w/w) | 0.35 | 0.40 | 0.42 |
| Rare earth content (% w/w) | 1.94 | 2.06 | 1.84 |
| Phosphorus content (% w/w) | 0.42 | 0.22 | 0.62 |
| ZSM-5 content (% w/w) | 0.00 | 0.00 | 1.50 |

CFs that modify the FCC-SIM™ reaction model follow an experimental design for the assessment of catalysts in a laboratory unit or pilot plant (FCC-SIM Technology Manual Version 2002. KBC PROFIMATICS. 2004).

To estimate the CF factors for the CDB/FCC-SIM™ model, the following strategy was adopted: Firstly, a standard condition was adopted (Table VI) and the same database was repeated 9 times.

**Variable**

Table VI - Arbitrated standard operational condition.

| RXT (Risers 1 and 2) | 533 | Naphtha riser flow rate ($m_3/d$) | 600 |
|---|---|---|---|
| DPT | 707 | d20/4 feed | 0.936 |
| DilPT | 710 | Basic nitrogen of the feed (ppm) | 1100 |
| CFT | 255 | Catalyst replacement (t/d) | 5 |
| Blower air flow rate (t/h) | 192 | Sodium content in Ecat (ppm) | 4300 |
| Feed flow rate ($m^3/d$) | 7400 | Iron content in Ecat (ppm) | 4200 |
| DD GOP flow rate ($m^3/d$) | 6000 | Vanadium content in Ecat (ppm) | 1150 |
| GOPK flow rate ($m^3/d$) | 800 | Nickel content in Ecat (ppm) | 700 |

Arbitrated standard condition:

- Two datasets; only replacements 4 and 6 t/d were changed;

- One of the sets adopted 545°C RXTs;

- One of the sets adopted: CFT of 200 °C, a vanadium content of 500 ppm, a nickel content of 400 ppm, and a replacement of 6 t/d;

- One of the sets adopted: CFT of 200 °C, a vanadium content of 500 ppm, a nickel content of 400 ppm, and a replacement of 4 t/d;

- One of the sets adopted: a vanadium content of 500 ppm, a nickel content of 400 ppm;

- One of the sets adopted: a vanadium content of 1800 ppm, a nickel content of 1000 ppm;

- One of the sets adopted: a vanadium content of 1800 ppm, a nickel content of 1000 ppm;

For all the conditions described above, the yields and CFs were estimated for the catalysts whose properties are listed in Table V. The CF factors were converted into a file and used directly in the simulation within the CDB/FCC-SIM™ module.

Table VII shows the yield deltas obtained both in the industrial assessment of catalysts and in the prediction of the developed Hybrid MLP model, wherein the assessed catalytic systems are:

- SIRIUS 2727 base catalyst (August 19, 2015 to May 4, 2016): 50% VEGA 2640 + 50% OPAL SC LRT;
- Catalyst assessed SIRIUS 2147 (January 11, 2017 to July 25, 2018): 25% VEGA 2742 + 75% OPAL SC LRT.

Table III - Comparison of the catalytic performance in an industrial assessment of SIRIUS 2147 catalyst relative o SIRIUS 2727, and equivalent prediction of the hybrid MLP model.

| Assessment | Conventional catalyst assessment | | Hybrid MLP model | | |
|---|---|---|---|---|---|
| Yield | Group | Neural networks MLP[1] | Model MLP H.[2] | Model MLP H.[1] | CDB/FCC-Yes |
| Database used | | 87 | 4974 | 87 | |
| GC | -0.41 | +0.003 | + 0.06 | + 0.06 | -0.04 |
| H2 | +0.012 | +0.014 | +0.008 | 0.00 | +0.002 |
| GLP | + 1.32 | + 1.08 | + 0.32 | + 0.19 | + 0.69 |
| Propene | + 0.21 | + 0.33 | + 0.20 | + 0.09 | + 0.14 |
| NC | + 1.58 | + 0.32 | + 0.30 | + 0.10 | + 0.23 |
| LCO | -1.88 | -0.59 | -0.33 | -0.22 | -0.28 |
| OD | -1.09 | -0.89 | -0.55 | -0.34 | -0.63 |
| Coke | + 0.39 | + 0.06 | + 0.21 | + 0.21 | + 0.02 |
| Delta coke | + 4.6% | -0.2% | - | - | + 1.2% |

The performance assessment of SIRIUS 2147 catalyst relative to SIRIUS 2727 catalyst was carried out by two different analyses: assessment by similar groups and neural networks (Table VII). Industrial assessment of the catalytic systems has shown that performance of the SIRIUS 2147 catalyst is superior to that of SIRIUS 2727 catalyst for the yields of LPG, propene and naphtha, with a reduced yield of LCO and decanted oil (OD). Predictions of the Hybrid MLP model applied to the catalytic systems has shown agreement with the industrial assessment for an increased naphtha yield with reduction in LCO and bottoms.

Table VIII shows the yield deltas obtained both in the industrial assessment of catalysts and in the prediction of the developed Hybrid MLP model, wherein the assessed catalytic systems are:

- SIRIUS 2147 base catalyst (January 11, 2017 to July 25, 2018): 25% VEGA 2742 + 75% OPAL SC LRT;
- Catalyst assessed SIRIUS ZOOM 2176 (January 2, 2019 to November 17, 2021): 24,6% VEGA 2742 + 72,19% OPAL SC LRT + 3,75% ZOOM.

Table IV - Comparison of catalytic performance in industrial assessment of the SIRIUS ZOOM 2176 catalyst in relation to SIRIUS 2147, and equivalent prediction of the hybrid MLP model.

| Assessment | PETROBRAS Catalyst Assessment Standard | | Hybrid MLP model | | |
|---|---|---|---|---|---|
| Yield | Neural networks MLP[1] | GOP model Simplified ZSM-5 1.0 | Model MLP H.[2] | Model MLP H.[1] | CDB/FCC-Yes |
| Database used | 140 | | 4974 | 140 | |
| GC | -0.26 | -0.13 | -0.22 | -0.13 | -0.48 |
| H2 | -0.002 | - | -0.007 | - | -0.001 |
| GLP | + 4.08 | + 3.26 | + 4.02 | + 4.21 | + 3.82 |
| Propene | + 1.72 | + 2.18 | + 1.69 | + 2.09 | + 1.67 |
| NC | -2.80 | -2.89 | -3.21 | -3.28 | -3.59 |
| LCO | + 0.16 | -0.14 | + 0.23 | + 0.46 | + 1.41 |
| OD | -1.41 | -0.11 | -1.01 | -1.49 | -1.15 |
| Coke | + 0.22 | 0.00 | + 0.19 | + 0.24 | -0.01 |
| Delta coke | -0.4% | -11.8% | - | - | -3.31% |

Assessment of SIRIUS 2147 catalyst performance in relation to SIRIUS ZOOM 2176 catalyst was carried out using only neural networks. Such an assessment was complemented with the prediction of yield using the Simplified GOP ZSM-5 1.0 Model (Table VIII). The Simplified GOP ZSM-5 1.0 Model was developed to predict the performance of ZSM-5 zeolite under various operational conditions and is based on a wide range of tests carried out in a pilot plant - DCR (PINHO, A. R. Maximização de Olefinas Luzs com Gasóleo Pesado de Vacuo. Rio de Janeiro: PETROBRAS. CENPES. PDAB. TFCC, 2012. 15 f. Technical Report (RT TFCC No. 001/2012)). Industrial assessment of the catalytic systems has shown that performance of the SIRIUS ZOOM 2176 catalyst is greater than that of the SIRIUS 2727 catalyst for LPG and propene yields, with a reduced yield of fuel gas, cracked naphtha and, unexpectedly, a reduced yield of decanted oil. Predictions of the Hybrid MLP model applied to the catalytic systems has shown agreement with the industrial assessment and the Simplified GOP ZSM-5 1.0 Model, exhibiting an equivalent performance profile.

Modeling of REPLAN U-220 catalytic cracking unit as a function of its operational conditions and the catalytic systems used was carried out in accordance with the method proposed by the present invention. This approach seeks to take advantage of both methods, using physical modeling to capture prior knowledge and existing physical relationships, while machine learning is used to deal with the complexity and uncertainty of the systems, hence increasing the accuracy, interpretation and robustness of the models.

Modeling was carried out according to the following steps: 1) Pre-training using simulated data from the FCC-SIM™ simulator, which takes into account physical principles such as material and energy balances and the effects of operational variables on unit performance;

2) Incorporation of operational data, catalyst system quality, and product yields from the U-220 unit for the period from January 2011 to June 2022. The trained models were assessed by RMSE (*Root Mean Square Error*) and its coefficient of correlation ($R^2$), with minor errors (low RMSE values) and high $R^2$ values being observed for the pre-training model, the best model being for a number of 50 neurons in the two inner layers. This strategy was used to re-estimate the MLP model parameters based on historical data from the U-220 unit. At this stage, 10 iterations were performed to obtain the RMSE and $R^2$ of the Hybrid MLP model of the present invention. The best model, which exhibited the lowest individual yield errors and the highest $R^2$ value was used in the prediction and assessment of the method proposed herein.

The Hybrid MLP model was compared across four catalyst reformulations of the U-220 unit and against the industrial assessments performed in accordance with the PETROBRAS standard for industrial catalyst assessment. For the Hybrid MLP model, the only catalyst information provided was the properties of fresh catalysts. The Hybrid MLP model was assessed in three different ways, one being the prediction of catalyst performance for the entire database, another being the prediction for the dataset during the catalyst usage period in the unit, and another considering the CDB/FCC-SIM™ model adapted with the CFs predicted by the model. All comparisons correctly predicted the trend to convert the bottoms both by the industrial assessment and by the Hybrid MLP model of the present invention. It is then possible to state that the Hybrid MLP model performed well in predicting the improvement in TOPAZ technology (FCC catalyst manufacturing technology) and the use of a ZSM-5 based additive, correctly indicating the performance trend of the main yields: LPG, propene, naphtha, LCO, and bottoms.

Therefore, results from the Hybrid MLP model provided an estimation of the FCC unit performance by informing only the properties of the fresh catalyst and the operational conditions, with extremely reduced costs when compared to other current alternatives. The proposed method will be implemented to PETROBRAS *Digital Twin* FCC model allowing for an indication of the best formulation based on the market demand for products.

**Claims**

1.  A method for predicting the performance of a fluid catalytic cracking (FCC) unit, **characterized in that** it comprises:

    pre-training a predictive model using a simulated database (BD_FCCSIM) that represents various operational scenarios of a FCC unit;
    refining the predictive model with a historical database (BD_Histórica) of the actual operation of a FCC unit including fresh catalyst and Ecat parameters as well as process parameters with their respective yields over a ten-year period;
    fitting and setting the model to predict FCC unit yields and catalyst characteristics based on the operational conditions and properties of the catalysts used;
    validating the model based on error and correlation metrics, to ensure its application in simulated and actual scenarios;
    implementing the predictive model in a monitoring system to adjust the operations of the FCC unit in accordance with changes in the operational conditions.

2.  The method according to claim 1, **characterized in that** the simulated database (BD_FCCSIM) and the historical database (BD_Histórica) include input variables such as ZSM-5 zeolite content, reaction temperature (RXT), dense phase temperature (DPT), dilute phase temperature (DilPT), combined feed temperature (CFT), feed flow rate ($F_{Feed}$), GOP flow rate $F_{GOP}$), GOPK flow rate (VGopk), naphtha riser flow rate (NRF), 20/4 density of the feed ($d20_{Feed}$), 20/4 density of the coke gas oil (d20Gopk), basic nitrogen of the feed ($BN_{Feed}$), specific area of the catalyst (VCatSA), deactivated catalyst specific area (VCatSAD), catalyst pore volume (VCatPV), catalyst bulk density (VCatBD), catalyst unit cell size (VCatUCS), catalyst accessibility index (VCatAI), catalyst alumina content (VCatAl), sodium content (VCatNa), iron content (VCatFe), rare earth content (VCatRE), phosphorus content (VCatP), vanadium content in the Ecat (ECatV), nickel content in the Ecat (ECatNi), phosphorus content in the Ecat (ECatP), coke content in the Ecat (ECatC), activity of the deactivated catalyst (VCatMAT), Ecat activity (ECatMAT), and air flow rate of the blower (AirBlow).

3.  The method according to claim 1, **characterized in that** the simulated database (BD_FCCSIM) is generated through a factorial simulation design that generates multiple scenarios by varying the catalyst conditions and process parameters.

4.  The method according to claim 1, **characterized in that** the pre-training step uses a factorial design carried out over two different periods with two distinct calibrations, such that the chosen periods have the same base catalyst, wherein one period uses a ZSM-5 additive and the other does not.

5.  The method according to claim 1, **characterized in that** the historical database (BD_Histórica) comprises actual operational data from the FCC unit over an extended period of time, including information on process conditions and parameters of fresh and equilibrium catalysts (Ecat).

6.  The method according to claim 1, **characterized in that** the historical database (BD_Histórica) contains product yield data including: fuel gas, LPG, cracked naphtha, LCO, decanted oil, coke, and gas chromatography data (hydrogen and C1 to C4).

7.  The method according to claim 1, **characterized in that** the predictive model fitting includes setting a multilayer perceptron (MLP) neural network with one input layer, two hidden layers, and one output layer, the input layer comprising operational and catalyst variables.

8.  The method according to claim 7, **characterized in that** the hidden layers of the predictive model are set to include between 50 and 200 neurons per layer, with activation functions of the *ReLU* and *tanh* types, by applying normalized weight initializations.

9. The method according to claim 1, **characterized in that** the predictive model validation includes:

calculation of the root mean square error (RMSE) to assess the accuracy of the model's predictions relative to the actual data; and
calculation of the coefficient of correlation ($R^2$) between the values predicted by the model and the actual values achieved in testing and validation scenarios.

10. The method according to claim 1, **characterized in that** the model validation includes selecting a model setting that minimizes the RMSE and maximizes the $R^2$ in the training, testing, and validation datasets.

11. The method according to claim 1, **characterized in that** the monitoring system uses the model predictions to adjust operational variables of the FCC unit, such as reaction temperature, feed flow rate, and catalyst replacement rate, in accordance with the operational conditions and characteristics of the catalyst in use.

12. The method according to claim 1, **characterized in that** implementation of the predictive model in the monitoring system allows for the storage and processing of the predicted Catalyst Factors (CFs) for each catalyst, which are used to adjust the reaction factors, allowing the prediction and optimization of the FCC unit yields.
The method according to claim 1, **characterized in that** the monitoring system with the predictive model is connected to the digital twin of the FCC unit, enabling future scenario simulations and operational adjustments based on the catalyst performance and yield predictions.

FIGURE 1

FIGURE 2

Hydrogen (% by mass)

Legend:
- Training prediction
- Actual data
- Test prediction
- Actual data
- Validation prediction
- Actual data

Y-axis (Hydrogen (% by mass)): 0.16, 0.14, 0.12, 0.10, 0.08, 0.06, 0.04, 0.02

X-axis: 2012, 2014, 2016, 2018, 2020, 2022

FIGURE 3

FIGURE 4

FIGURE 5

EP 4 768 556 A1

FIGURE 6

FIGURE 7

FIGURE 8

FIGURE 9

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/004356 A1 (ZHAO HONG [US] ET AL) 4 January 2024 (2024-01-04) * paragraphs [0043], [0077], [0082], [0083], [0089]; claims 1-3; figures 6A,6B * | 1-13 | INV. C10G11/18 |
| A | LI TIANYUE ET AL: "A bilevel data-driven framework for robust optimization under uncertainty - applied to fluid catalytic cracking unit", COMPUTERS & CHEMICAL ENGINEERING, PERGAMON PRESS, OXFORD, GB, vol. 166, 11 September 2022 (2022-09-11), XP087186793, ISSN: 0098-1354, DOI: 10.1016/J.COMPCHEMENG.2022.107989 [retrieved on 2022-09-11] * 4.2 Construction of DDRO model; page 7; figure 4 * | 1-13 | |
| A | US 2020/103838 A1 (BERTINETTI MARK [AU] ET AL) 2 April 2020 (2020-04-02) * paragraphs [0063], [0066], [0067] * | 1-13 | TECHNICAL FIELDS SEARCHED (IPC) C10G G16C |

The present search report has been drawn up for all claims

| Place of search | Date of completion of the search | Examiner |
|---|---|---|
| The Hague | 27 January 2026 | Chau, Thoi Dai |

EPO FORM 1503 03.82 (P04C01)

## ANNEX TO THE EUROPEAN SEARCH REPORT
## ON EUROPEAN PATENT APPLICATION NO.

EP 25 20 1539

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2026

| Patent document cited in search report | | Publication date | Patent family member(s) | | Publication date |
|---|---|---|---|---|---|
| US 2024004356 | A1 | 04-01-2024 | US | 2024004356 A1 | 04-01-2024 |
| | | | WO | 2024006873 A1 | 04-01-2024 |
| US 2020103838 | A1 | 02-04-2020 | AU | 2019350984 A1 | 29-04-2021 |
| | | | AU | 2022275387 A1 | 05-01-2023 |
| | | | AU | 2025200676 A1 | 20-02-2025 |
| | | | EP | 3857321 A1 | 04-08-2021 |
| | | | US | 2020103838 A1 | 02-04-2020 |
| | | | WO | 2020069247 A1 | 02-04-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11676061 B2
- US 10838412 B2
- US 11987758 B2
- US 11669063 B2

**Non-patent literature cited in the description**

- **BASTIANI, R. et al.** 76f Technical Communication (CT TFCC No. 007/2011). *Padrão de Avaliação Industrial de Catalisadores. Rio de Janeiro: PETROBRAS. CENPES. PDAB. TFCC*, 2011
- *FCC-SIM Technology Manual Version*, 2002
- *KBC PROFIMATICS.*, 2004
- **PINHO, A. R.** 15 f. Technical Report (RT TFCC No. 001/2012). *Maximização de Olefinas Luzs com Gasóleo Pesado de Vacuo. Rio de Janeiro: PETROBRAS. CENPES. PDAB. TFCC*, 2012